# EUROPEAN PATENT SPECIFICATION

(11) **EP 4 166 146 B1**
(45) Date of publication and mention of the grant of the patent: **21.02.2024**
(21) Application number: 22461623.5
(22) Date of filing: 17.10.2022
(51) Int. Cl.: A61K 31/795, A61P 31/14

(54) **PAMPS-PAAU COPOLYMERS FOR USE IN THE TREATMENT OR PROPHYLAXIS OF INFECTION CAUSED BY THE ZIKA VIRUS**
PAMPS-PAAU COPOLYMERE ZUR BEHANDLUNG ODER PROPHYLAXE VON DURCH DAS ZIKA VIRUS VERURSACHTEN INFEKTIONEN
COPOLYMÈRES DE PAMPS-PAAU DESTINÉS À ÊTRE UTILISÉS DANS LE TRAITEMENT OU LA PROPHYLAXIE D'UNE INFECTION PROVOQUÉE PAR LE VIRUS ZIKA

(30) Priority: 15.10.2021 PL 43922621
(43) Date of publication of application: 19.04.2023
(73) Proprietor: UNIWERSYTET JAGIELLONSKI, 31-007 Kraków (PL)
(72) Inventor: Nowakowska, Maria, 30-433 Kraków (PL); Pyrc, Krzysztof, 30-433 Kraków (PL); Szczubialka, Krzysztof, 32-442 Krzywaczka (PL); Botwina, Pawel, 30-150 Kraków (PL); Obloza, Magdalena, 30-382 Kraków (PL)
(74) Representative: Witek, Rafal

(56) References cited:
- WO-A1-2017/190193
- WO-A1-2021/201705
- TAN CHEE WAH ET AL: "Polysulfonate suramin inhibits Zika virus infection", ANTIVIRAL RESEARCH, ELSEVIER BV, NL, vol. 143, 27 April 2017 (2017-04-27), pages 186-194, XP085032225, ISSN: 0166-3542, DOI: 10.1016/J.ANTIVIRAL.2017.04.017
- TAN CHEE WAH ET AL: "In Vitro Inhibition of Zika Virus Replication with Poly(Sodium 4-Styrenesulfonate)", VIRUSES, vol. 12, no. 9, 23 August 2020 (2020-08-23), page 926, XP055823235, DOI: 10.3390/v12090926
- MOHAN P ET AL: "SULFONIC ACID POLYMERS AS A NEW CLASS OF HUMAN IMMUNODEFICIENCY VIRUS INHIBITORS", ANTIVIRAL RESEARCH, ELSEVIER BV, NL, vol. 18, 1 January 1992 (1992-01-01), pages 139-150, XP000995720, ISSN: 0166-3542, DOI: 10.1016/0166-3542(92)90034-3
- IKEDA S ET AL: "IN VITRO AND IN VIVO INHIBITION OF ORTHO- AND PARAMYXOVIRUS INFECTIONS BY A NEW CLASS OF SULFONIC ACID POLYMERS INTERACTING WITH VIRUS-CELL BINDING AND/OR FUSION", ANTIMICROBIAL AGENTS AND CHEMOTHERAPY, AMERICAN SOCIETY FOR MICROBIOLOGY, US, vol. 38, no. 2, 1 February 1994 (1994-02-01), pages 256-259, XP000996063, ISSN: 0066-4804
- BOTWINA PAWEL ET AL: "Self-Organized Nanoparticles of Random and Block Copolymers of Sodium 2-(Acrylamido)-2-methyl-1-propanesulfonate and Sodium 11-(Acrylamido)undecanoate as Safe and Effective Zika Virus Inhibitors", PHARMACEUTICS, vol. 14, no. 2, 27 January 2022 (2022-01-27), page 309, XP93023183, DOI: 10.3390/pharmaceutics14020309

## Description

The invention relates to PAMPS-PAaU copolymers for use in the treatment and/or prevention of Zika virus (ZIKV) infection.

An inhibitor of RNA virus infection is known from European patent application EP2444095A1. The inhibitor of RNA virus infection contains a compound inhibiting RNA virus infection which is a linear polymer. Its macromolecules have been substituted with single aromatic rings, such as phenyl, or condensed rings, such as naphthyl, forming side chains. The polymer described in this application is either crosslinked and thus insoluble, or immobilised on an insoluble support such as talc, bentonite, silica, vermiculite and perlite, among others. Such a polymer can thus be used to decontaminate everyday objects such as tatami mats, carpets, furniture, car interiors, kitchen items, wallpaper, fabrics, etc. Among the potential uses as a medicinal substance, only external applications (medicines, medical products and cosmetics) are listed.

Prior art document (Tan Chee Wah, "Polysulfonate suramin inhibits Zika virus infection", ANTIVIRAL RESEARCH, ELSEVIER BV, NL, vol. 143, 27 April 2017) discloses the inhibition of Zika virus by polysulphonated suramin. Prior art document (Tan Chee Wah, "In Vitro inhibition of Zika Virus Replication with Poly(Sodium 4-Styrenesulfonate)", VIRUSES, vol. 12, no. 9, 23 August 2020) discloses the in vitro inhibition of Zika Virus replication with Poly(Sodium 4-Styrenesulfonate). Prior art document (WO 2021/201705) discloses the use of poly(sodium 4-styrene sulfonate) for treating and/or prophylaxy of the infection caused by Zika virus. Prior art document (WO 2017/190193) discloses methods for preventing transmission of Zika virus comprising the topical administration of a macromolecule dendrimer of 1 to 8 generations with one or more sulfonic acid- or sulfonate-containing moieties attached to one or more surface groups of the dendrimer.

From the Polish patent application P.433443, the compound PSSNa (sodium salt of poly(styrene 4-sulfonate) for use in the treatment and/or prevention of ZIKV infection is known. Results of functional tests have shown that PSSNa acts through direct interaction with the Zika virus. However, a high molecular weight polymer is necessary for satisfactory efficacy. PSSNa 3160 kDa reduced the TCID₅₀ value by almost 3 log, while PSSNa 1.1 kDa reduced the value only by 0.5 log. Also, a direct interaction of PSSNa with the receptor was excluded, since no decrease in ZIKV replication was observed in a cell protection assay in which the polymers were incubated with cells prior to infection. No decrease in ZIKV virion number was observed in cells treated with PSSNa after the initial stages of virus replication. Therefore, it can be assumed that the antiviral effect of PSSNa is mainly due to its direct interaction with ZIKV particles, preventing the virus from attaching to host cells.

Zika virus (ZIKV) is an RNA virus belonging to the *Flaviviridae* family. It is closely related to other flaviviruses, such as West Nile virus (WNV), dengue virus (DENV), Japanese encephalitis virus (JEV) and yellow fever virus (YFV)¹. ZIKV is an arbovirus that requires an *Aedes* mosquito vector for spread², but infection can also occur through direct contact, including sexual contact³. ZIKV was first isolated in 1947 from a macaque specimen⁴, while a year later the virus was found in mosquitoes of *Aedes* species living in the Zika Forest in Uganda⁴. The first three cases of human infection were reported in 1954 in Nigeria⁵. In 2007, the island of Yap (Micronesia) experienced a sudden outbreak of Zika fever affecting 73% of the island's populations. The next outbreak occurred in 2013-2014 in Polynesia affecting 32 000 people^{7,8}. The third, and most severe outbreak to date, occurred in Brazil in 2015 affecting 1.3 million people⁹⁻¹¹. In 2016, the first cases of the disease transmitted by local mosquitoes were reported in the USA, and already by September 2016, 1 624 cases of the disease had been recorded in the EU. The first case of Zika fever in Poland was found in 2016 in a person who had returned from a trip to the Dominican Republic and Colombia. There is concern that progressing climate change could also cause ZIKV to spread to temperate zone regions, including Europe and North America. Although in most cases of infection the disease has a relatively mild course characterised by fever, rash and joint pain, the sequelae can be very serious.

ZIKV is a neurotropic virus that can cause serious neurological complications such as Guillain-Barre syndrome (GBS) or congenital Zika syndrome (CZS)¹² in foetuses, associated with mother-to-foetus virus transmission. ZIKV infection is therefore particularly dangerous for pregnant women. Such infection can cause miscarriage, premature birth and stillbirth, as well as damage to the nervous system of new-borns. The most common birth defects and disabilities associated with CZS include microcephaly, brain tissue damage, posterior eye damage, macular scarring, congenital contractures such as clubfoot or arthrogryposis, as well as hypertonia limiting the body movement of a neonate^{9,12-15}. World Health Organisation (WHO) data from 2019 indicate that ZIKV transmission has been recorded in 87 countries and territories. As ZIKV has spread worldwide in a relatively short period of time, with numerous studies revealing further adverse and life-threatening effects of the disease caused by this virus, the WHO has classified ZIKV as a pathogen of public health emergency of concern. In 2016, the WHO declared a global Zika virus emergency, while the prestigious journal Nature identified research into the Zika fever epidemic as one of the most important scientific developments of 2016.

To date, no vaccine or drug for Zika fever has been developed and treatment is only supportive. Although intensive research is currently underway to develop a vaccine and drugs, it is still at an early stage of development. A factor hindering the development of a drug for Zika fever is related to the fact that ZIKV mutates extremely rapidly. This observation points to the need to develop a broad-spectrum drug for ZIKV infection. Potential candidates for such a drug could be polymers containing multiple repeating structural units in their macromolecules. These facilitate polyvalent interactions with viral proteins resulting in inactivation of that protein and/or the host cell membrane, thus providing protection against infection. As noted above, despite many years of research, there is still no approved drug against ZIKV.

According to the invention, PAMPS means polymer of sodium salt of 2-acrylamido-2-methyl-1-propanesulfonic acid, while PAaU means polymer of sodium 11-(acrylamido)undecanoate. According to the invention, AMPS means a single mer of sodium salt of 2-acrylamido-2-methyl-1-propanesulfonic acid, while AaU means a single mer of sodium 11-(acrylamido)undecanoate. According to the invention, by diblock copolymer is meant a copolymer containing two large blocks of mers, one block being PAMPS and the other block being PAaU (PAMPS-block-PAaU). According to the invention, a statistical copolymer is to be understood as a copolymer in which the mers of AMPS and AaU (PAMPS/PAaU copolymer) are randomly distributed along the chain according to known statistical laws.

The aim of the present invention was to develop a novel inhibitor to prevent Zika virus infection, alleviate its symptoms and reduce the risk of complications. It was hypothesised that the most effective would be self-organising in aqueous solutions diblock amphiphilic PAMPS-block-PAaU copolymers (PAMPS-b-PAaU) and statistical amphiphilic PAMPS/PAaU copolymers, which would effectively inhibit viral replication *in vitro.* At the same time, the polymers used would have low toxicity towards monkey kidney cells (Vero PZH), which are susceptible to Zika virus infection.

Unexpectedly, all the above-mentioned technical problems have been solved by the present invention.

The subject of the invention are PAMPS/PAaU copolymers of formula: 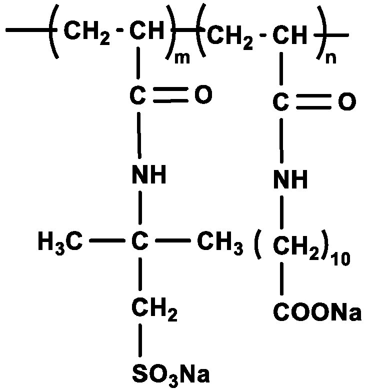 wherein,
m denotes the length of the PAMPS block, namely the number of AMPS mers and is 40-170,
n denotes the length of the PAaU block, namely the number of AaU mers and is 3-50, for use in the treatment and/or prevention of Zika virus infection.

Preferably, the copolymers of formula: 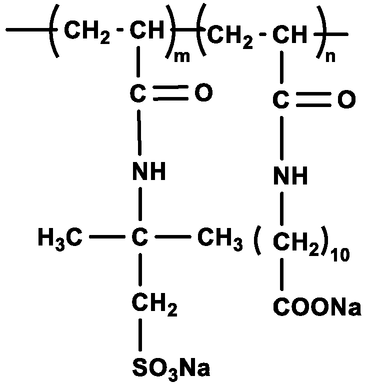 are characterised in that
m is 50-75 and n is 12-40.

Preferably, the copolymers are selected from the group comprising the block copolymers PAMPS₇₅-b-PAaU₁₂, PAMPS₇₅-b-PAaU₂₈, PAMPS₇₅-b-PAaU₃₉, or the selected copolymer is the statistical copolymer P(AMPS₅₀/AaU₅₀).

Preferably, the copolymers are characterised in that the effective concentration of either the block copolymer or the statistical copolymer inhibiting Zika virus replication is 5-25 µg/mL.

Preferably, the copolymers are characterised in that the effective concentration of the PAMPS₇₅-b-PAaU₃₉ block copolymer inhibiting Zika virus replication is not less than 5 µg/mL.

The PAMPS₇₅-b-PAaU₃₉ block copolymers with well-defined structure and composition can be obtained by modern controlled radical polymerisation method (RAFT) as described in the literature ¹⁶. The statistical copolymer P(AMPS₅₀/AaU₅₀) can be obtained by classical free radical polymerisation. The copolymers undergo spontaneous self-organisation in aqueous solution with the formation of well-defined, surface-charged, core-shell nanoparticles/micelles. High zeta potential values provide stability of their dispersion in aqueous media. The micelles interact with receptors present on the cell membrane blocking their interaction with ZIKV particles preventing/reducing infection. The copolymers have very low toxicity to Vero cells.

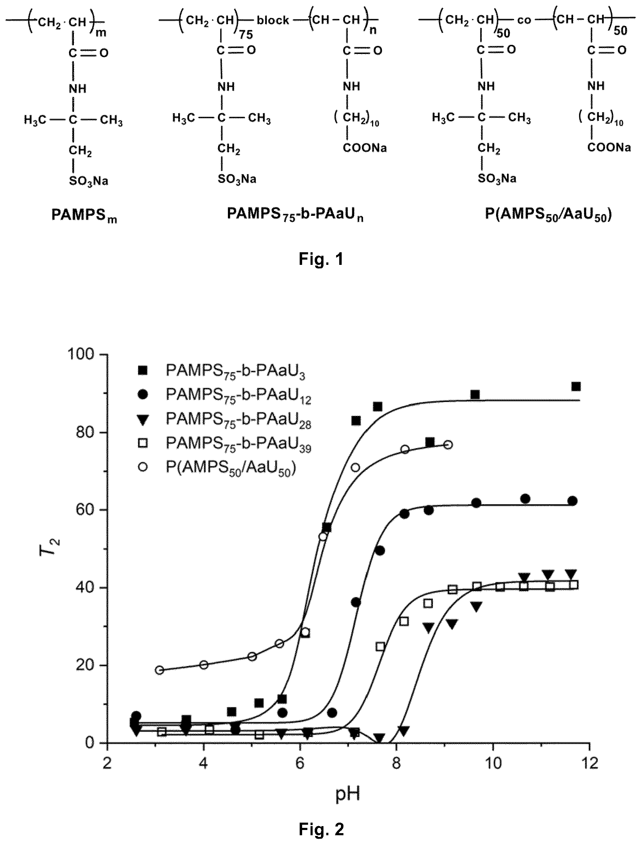

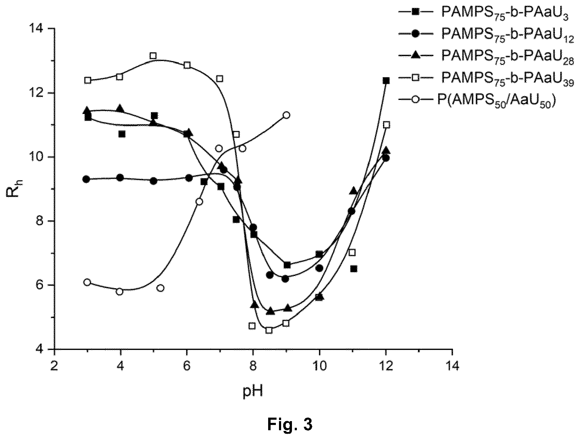

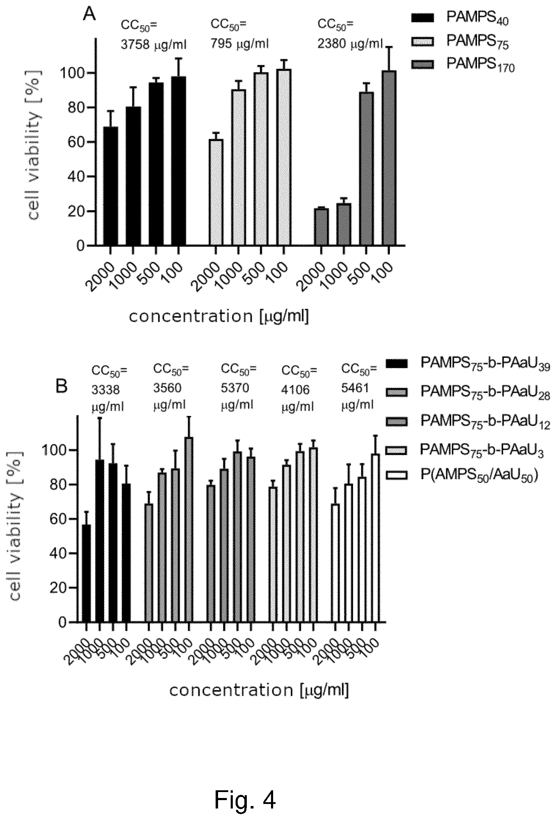

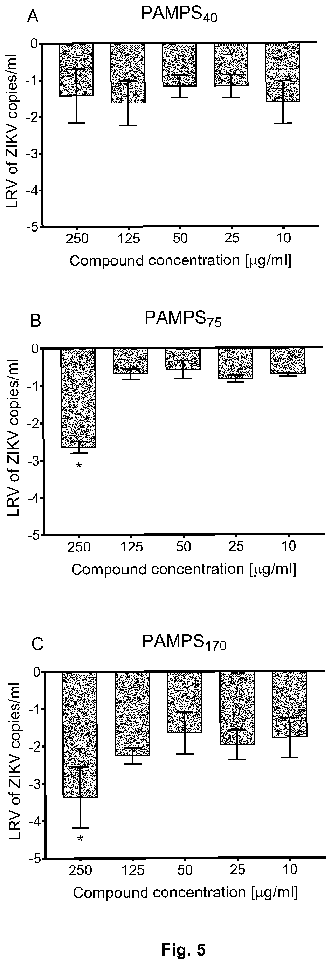

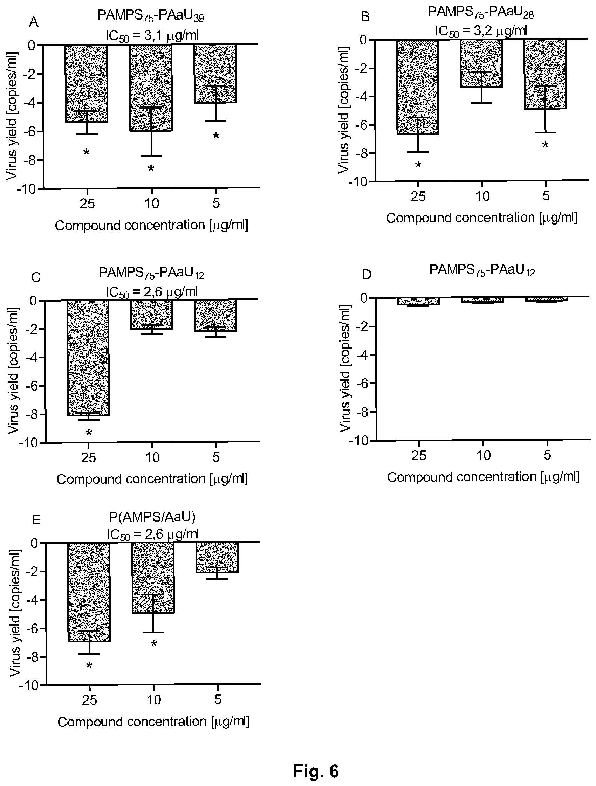

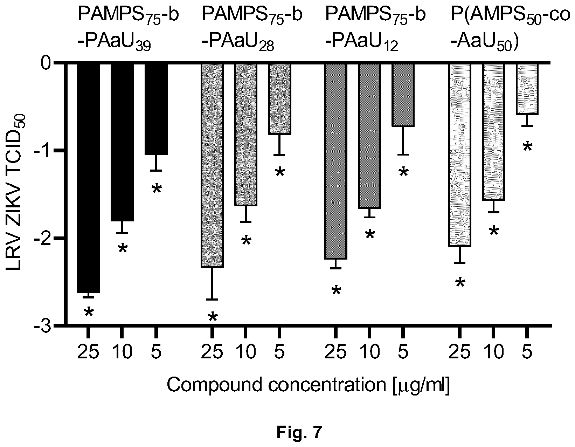

The subject of the invention in its embodiments is illustrated in figures, of which: fig. 1 shows the structures of PAMPS homopolymer, diblock copolymers PAMPS₇₅-b-PAaUₙ and statistical copolymer P(AMPS₅₀/AaU₅₀), fig.2 shows the results of ¹H NMR spin-spin relaxation time measurements for protons of side chain methyl group of AaU mers (signal at 1.25 ppm) as a function of pH, confirming the process of self-organisation of the tested copolymers in aqueous medium, leading to the formation of either inter- or intramolecular micelles (depending on pH), fig.3 shows changes in hydrodynamic radius of formed nanoparticles/micelles in aqueous medium as a function of pH, as determined by dynamic light scattering (DLS) technique, fig.4 shows the results of cytotoxicity measurements for PAMPSₘ homopolymers, PAMPS₇₅-b-PAaUₙ block copolymers and P(AMPS₅₀/AaU₅₀) statistical copolymer at concentrations of 2000, 1000, 500 and 100 µg/mL as determined by XTT assay using Vero cells, fig.5 shows the mean values of the logarithm of the viral RNA copy number with standard deviations (error bars), representing the inhibition of ZIKV replication cycle in Vero cells by PAMPSₘ homopolymers at five different concentrations of 250, 125, 50, 25, 10 µg/mL, fig. 6 shows the mean values of the logarithm of the viral RNA copy number with standard deviations (error bars), representing the block copolymers PAMPS₇₅-b-PAaUₙ and the statistical copolymer P(AMPS₅₀/AaU₅₀) at three different concentrations of 25, 10, 5 µg/mL, fig. 7 shows the titration results for the supernatant taken from Vero cells infected in the absence and presence of the block copolymer PAMPS₇₅-b-PAaUₙ and the statistical copolymer P(AMPS₅₀/AaU₅₀) at different concentrations, fig. 8 shows the antiviral activity of the tested block copolymers PAMPS₇₅-b-PAaUₙ and statistical copolymer P(AMPS₅₀/AaU₅₀) at different stages of the ZIKV infection cycle in Vero cells, the tests performed being: A) virus inactivation assay; B) cell protection assay; C) virus entry assay; D) late stages of virus replication assay, fig. 9 shows fluorescence images presenting inhibition of ZIKV infection of Vero cells by PAMPS₇₅-b-PAaU₃₉ block copolymer and P(AMPS₅₀/AaU₅₀) statistical copolymer obtained using fluorescence confocal microscopy, fig.10 shows the results of observations using fluorescence confocal microscopy confirming that the PAMPS₇₅-b-PAaU₃₉ copolymer causes inhibition of the ZIKV replication cycle in Vero cells as a result of blocking virus adhesion to the cell.

The results presented here suggest a high antiviral potential of the presented copolymers and justify proceeding to the next stages of research into use thereof in the treatment of infections caused by this virus. Their main advantages are the possibility of obtaining macromolecules of a given composition and molecular weight using modern techniques of controlled radical polymerisation, excellent water solubility, lack of toxicity at high doses, very low inhibitory concentration and a mechanism of action based on blocking the entry of the virus into the cell. In addition, the amphiphilic copolymers have the ability to self-organise in aqueous solutions to form stable, nanostructured micellar systems dependent on the pH of the aqueous solution.

All experiments were carried out in at least three replicates. Results were presented as mean values ± standard deviation (SD). The IC₅₀ concentration value was determined using the Graph Pad Prism 8.0 function. The statistical significance of the results obtained was assessed using the Student's t-test and a value of P<0.05 was considered significant.

### Example 1 - Synthesis of PAMPS homopolymers and PAMPS-PAaU copolymers.

PAMPSₘ homopolymers (m = 40, 75 and 170) and a series of PAMPS₇₅-b-PAaUₙ diblock copolymers (n=3, 12, 28 and 39) were synthesised by controlled radical polymerisation technique (RAFT) using the method described in the literature¹⁶. Synthesis of a macro chain transfer agent PAMPS-macro-CTA was performed to obtain PAMPS-b-PAaU block copolymers. The exemplary procedure to obtain the PAMPS-macro-CTA polymer is as follows: AMPS (25.0 g, 121 mmol) was neutralised with 60 mL of aqueous NaOH solution (4.81 g, 121 mmol), followed by adding 4-cyanopentanoic acid dithiobenzoate (CTA, 232 mg, 0.829 mmol) and the initiator, 4,4'-azobis(4-cyanopentanoic acid) (46.4 mg, 0.166 mmol). Argon was passed through the reaction mixture for 30 min. Polymerisation was carried out at 70°C for 4 h. The polymer was purified by dialysis into water for one week and isolated by lyophilisation (yield 23.2 g, monomer conversion of 83.9%).

Exemplary procedure for the synthesis of PAMPS-b-PAaU: PAMPS-macro-CTA (2.43 g, 0.14 mmol), AaU (2.06 g, 7.43 mmol), and the initiator 4,4'-azobis(4-cyanopentanoic acid) (4.63 mg, 0.0165 mmol) were dissolved in 13.5 mL of water. Argon was passed through the system for 30 min. Polymerisation was carried out at 70°C for 4 h. The diblock polymer was purified by dialysis against diluted NaOH solution (pH=8) for one week and isolated by lyophilisation (yield 3.52 g, monomer conversion 89.4%). Number average molar weight (Mn) and dispersity index (Mw/Mn) were determined by GPC chromatography measurements.

The statistical copolymer P(AMPS₅₀/AaU₅₀) was obtained by classical free radical polymerisation method. AMPS (4.14 g, 20 mmol) was neutralised with NaOH (0.89 g, 22 mmol) in 80 mL of methanol, 5.55 g (20 mmol) of AaU and 16 mg (0.1 mmol) of AIBN were added. The reaction mixture was degassed using a vacuum line for six freeze-vacuum-thaw cycles. Copolymerisation was carried out at 60°C for 12 h. The copolymer was purified by precipitation to a large excess of diethyl ether. The product was dissolved in water, dialysed against diluted NaOH solution (pH=8) for one week and isolated by lyophilisation. The composition of the copolymer was determined by ¹H NMR spectroscopy. The physicochemical characteristics of the obtained polymers are shown in Table 1.

**Table 1. Molecular weights and composition of the polymers**

| Polymer | Mₙ^{a} (·10⁴) | M_{w}^{a} (·10⁴) | M_{w}/Mₙ ^{a} | DP(AMPS) ^{b} | DP(AaU) ^{c} |
|---|---|---|---|---|---|
| PAMPS₄₀ | 0.94 | 1.21 | 1.28 | 40 | 0 |
| PAMPS₇₅ | 1.72 | 2.18 | 1.26 | 75 | 0 |
| PAMPS₁₇₀ | 3.93 | 4.65 | 1.18 | 170 | 0 |
| PAMPS₇₅-b-PAaU₃ | 1.85 | 2.61 | 1.41 | 75 | 3 |
| PAMPS₇₅-b-PAaU₁₂ | 3.20 | 4.53 | 1.42 | 75 | 12 |
| PAMPS₇₅-b-PAaU₂₈ | 5.05 | 6.72 | 1.33 | 75 | 28 |
| PAMPS₇₅-b-PAaU₃₉ | 7.63 | 9.36 | 1.23 | 75 | 39 |
| P(AMPS₅₀/AaU₅₀) | 4.38^{d} | 9.90^{d} | 2.26^{d} | 50^{e} | 50^{e} |

| | | | | | |
|---|---|---|---|---|---|
| ^{a} Determined by GPC in H₂O/CH₃CN (80/20, v/v) 0.1 M NaNOs solution, calibrated using PSSNa standards. ^{b} Degree of polymerisation of AMPS determined by the GPC method in H₂O/CH₃CN (80/20, v/v) 0.1 M NaNOs solution, calibration using PSSNa standards. ^{c} Degree of polymerisation of AaU determined by ¹H NMR in D₂O. ^{d} Determined by GPC - eluent: mixture of water and DMF (50/50, v/v), 50 mM LiBr. ^{e} mol%, determined from ¹H NMR measurements in D₂O at 95°C. | | | | | |

### Example 2 - Self-organisation of PAMPS-PAaU copolymers in aqueous media.

All polymers are water soluble. Due to the amphiphilic nature of the copolymers they self-organise in aqueous media with the formation of core-shell micelles. The self-organisation process is controlled by the acidity (pH) of the solution and can be monitored by observing/analysing the movement of the polymer chains. The spin-spin relaxation time (*T₂*) was measured by ¹H NMR as a function of pH. The dependence of the spin-spin relaxation time at a chemical shift of 1.25 ppm corresponds to the methyl group protons in the side chains of the PAaU block as a function of pH. In solutions with pH<8, the *T₂* value decreases due to reduced mobility in the formed hydrophobic cores of the polymer micelles.

These results were confirmed by measuring the dependence of the macromolecule hydrodynamic radius as a function of pH by dynamic light scattering (DLS).

### Results:

In low pH solutions (pH=3), the polymers form interpolymeric micelles having a hydrophobic PAaU core and a hydrophilic PAMPS shell. When the pH of the environment increases (pH>7), the polymers form intrapolymeric micelles, i.e., each micelle is made up of one polymer chain - one macromolecule (unimer micelles). In an environment with pH=7.4, the radius of the intrapolymer micelles was 8-11 nm. The values of hydrodynamic radius of the micelles reached minimum of about 5-7 nm for pH 8-9. A further increase in pH led to an increase in electrostatic repulsion forces between the same-sign ionic sulfonic side groups. At pH-12, these micelles dissociate completely and the polymer chain relaxes/extends. The behaviour of the statistical polymer P(AMPS₅₀/AaU₅₀) is different. This polymer forms intermolecular micelles even under low pH conditions. The values of hydrodynamic radius and zeta potential of the PAMPS-PAaU copolymer micelles under conditions similar to those of the biological experiments are presented in Table 2.

**Table 2. Hydrodynamic radius (Rₕ) values, surface areas of spherical polymeric nanoparticles/micelles, dispersity index and zeta potential values for the PAMPS-PAaU copolymers (c =1 mg/mL in PBS buffer, pH= 7.4, T = 25°C)**

| | PAMPS₇₅-b-PAaU₃ | PAMPS₇₅-b-PAaU₁₂ | PAMPS₇₅-b-PAaU₂₈ | PAMPS₇₅-b-PAaU₃₉ | P(AMPS₅₀/AaU₅₀) |
|---|---|---|---|---|---|
| Rₕ [nm] | 4.0 ± 0.1 | 4.8 ± 0.1 | 4.9 ± 0.5 | 11.6 ± 0.5 | 8.0 ± 2.6 |
| Micelle surface area [nm2] | 200.96 | 289.38 | 301.57 | 1 690.07 | 803.84 |
| PDI | 0.65 ± 0.12 | 0.94 ± 0.11 | 0.53 ± 0.06 | 0.32± 0.06 | 0.38 ± 0.07 |
| Zeta potential [mV] | -22.9 ± 1.3 | -24.3 ± 2.2 | -24.8 ± 2.5 | -25.7 ± 0.8 | -24.2 ± 1.2 |

The results presented above indicate that, under the biological experiment conditions (pH~7.4), macromolecules exist as negatively charged nanostructured micelles/nanoparticles with hydrodynamic radius in the range of 4-12 nm and zeta potential about -25 mV. The high zeta potential values of the polymeric micelles provide nanodispersion stability in aqueous environment. A comparison of the surface area value of the polymeric nanoparticles, calculated under the assumption that they are perfectly spherical objects, suggests that the exposure, and therefore the availability of ionic sulfonic groups to interact with the virus/cell, increases with increasing length of non-ionic blocks forming the core of the micellar structures.

### Example 3 - Polymer cytotoxicity study.

The effects of the homopolymers PAMPSₘ and the diblock copolymers PAMPS₇₅-b-PAaUₙ, as well as the statistical copolymer P(AMPS₅₀/AaU₅₀) on the survival of Vero cells were investigated. The cytotoxicity of the polymers was determined using the XTT viability assay kit (XTT Viability Assay Kit, Biological Industries, Israel). The assay allows quantification of cell metabolic activity. Only metabolically active living cells are capable of converting the tetrazolium substrate (2,3-bis-(2-methoxy-4-nitro-5-sulfophenyl)-(2H)-tetrazolium carboxanilide, XTT) into its colour derivative, the concentration of which, found from absorbance at 480 nm, correlates with the number of living cells and their viability. Permissive cell lines, i.e. monkey kidney cells (Vero PZH), were used for the experiment. Cells were cultured for 48 h in DMEM medium (Dulbecco's Modified Eagle's Medium) supplemented with 5% FBS (heat-inactivated foetal bovine serum), penicillin, streptomycin and gentamicin. Aqueous solutions of PAMPSₘ homopolymers, PAMPS₇₅-b-PAaUₙ diblock copolymers and P(AMPS₅₀/AaU₅₀) statistical copolymer were added to the medium at various concentrations. Water was added in the case of control samples. After incubating the cells in a 96-well plate for 3 days, the medium was removed and 100 µl of fresh medium and 20 µl of activated XTT were added to each well of the plate. After 2 h of incubation, supernatants were transferred to a transparent 96-well plate and absorbance at 480 nm was measured using a spectrophotometer in the standard way. The values obtained were normalised against the absorbance measured for control cells (without polymer), which were assigned 100% viability. Aqueous solutions of the homopolymers PAMPS₄₀, PAMPS₇₅, PAMPS₁₇₀, diblock copolymers: PAMPS₇₅-b-PAaU₃, PAMPS₇₅-b-PAaU₁₂, PAMPS₇₅-b-PAaU₂₈, PAMPS₇₅-b-PAaU₃₉, and the statistical copolymer P(AMPS₅₀/AaU₅₀) were tested.

### Results:

The results obtained indicate low cytotoxicity of the polymers at concentrations up to 500 µg/mL. Introduction of PAaU in the form of a block in the diblock polymers or statistically distributed sequences in the case of the statistical copolymer, further reduces the toxicity of the studied copolymers towards the tested cell line, leading to an increase in CC₅₀ value above 3 000 µg/mL.

### Example 4 - Effect of tested polymers on ZIKV infection - antiviral activity assays.

Antiviral activity assays for the copolymers were performed in a biosafety level two (BSL-2) laminar chamber using Zika virus (strain H/PF/2013). To determine the antiviral activity of the studied polymers against ZIKV, inhibition of ZIKV infection by the studied polymers was assayed as a function of their concentration. In this experiment, the tested polymer at a given concentration was present in the medium at each stage of the viral infection. The experiment used fully confluent Vero cells 48 h after seeding in a 96-well plate. The medium was removed and 100 µl of fresh medium supplemented with 5% FBS (heat-inactivated foetal bovine serum), penicillin, streptomycin, gentamicin and a solution of the tested polymer at various concentrations were added. Control samples did not contain polymers. 100 µl of ZIKV suspension (TCID₅₀=2000/mL) was added to the cells. The plates were incubated for 2 h at 37°C, then the cells were washed three times with PBS solution to remove unbound viral particles. Finally, 100 µL of the polymer solution in culture medium was added to each well and cells were incubated for 3 days at 37°C. After this time, supernatants were collected to assess the viral RNA copy number using quantitative Real Time PCR (RT-qPCR) or titration. Isolation of viral RNA was performed using a commercially available RNA isolation kit (Viral DNA/RNA Isolation Kit, A&A Biotechnology, Poland) according to the protocol provided by the manufacturer. RNA isolated in this way was subjected to reverse transcription (RT) and quantitative real-time PCR (qPCR). The RT-qPCR reaction was performed as follows:
3 µl of isolated viral RNA was transcribed into a cDNA template and amplified in a 10 µl reaction containing 1× GoScript^{™} RT Mix for 1-Step RT-qPCR, 1× GoTaq Probe qPCR Master Mix with dUTP, 100 nM of a specific probe labelled with 6-carboxyfluorescein (FAM) and 6-carboxytetramethylrhodamine (TAMRA) (5'-FAM-CGGCATACAGCATCAGGTGCATAGGAG-TAMRA-3'), and 450 nM of each primer (5'-TTGGTCATGATACTGCTGATTGC-3' and 5'-CCTTCCACAAAGTCCCTATTGC-3'). The reaction was performed in a thermocycler (CFX96 Touch^{™} Real-Time PCR Detection System, Bio-Rad) under the following conditions: 45°C for 15 min (reverse transcription), 95°C for 2 min, followed by 40 cycles of 15 seconds at 95°C and 30 seconds at 60°C.

Appropriate standards were prepared to assess the initial number of viral RNA molecules in the sample. The sequence fragment transcribed into cDNA was amplified using the primers described above. The DNA thus obtained was cloned into pTZ57R/T plasmid (Thermo Scientific, Poland) using InsTAclone PCR Cloning Kit (Thermo Scientific, Poland). Transformation of *E. coli* TOP10 strain (Life Technologies, Poland) was performed and the plasmid vector was amplified in the standard way. The plasmid was then purified using GeneJET Plasmid Miniprep Isolation Kit (Thermo Scientific, Poland) and linearised by digestion with *Kpn*I restriction enzyme. The concentration of linearised DNA was assessed by spectrophotometric measurement and the copy number per millilitre was calculated. Six 10-fold serial dilutions were used as templates for the real-time PCR reaction. The ability of the polymers to inhibit ZIKV proliferation was determined as the decrease in the logarithm of viral RNA copy number per millilitre of medium.

### Results:

Surprisingly, PAMPSₘ homopolymers exhibit poor antiviral activity against ZIKV. Only PAMPSₘ with the highest molecular weight shows an inhibitory effect, but only at relatively high concentrations (250 µg/mL). At lower concentrations of homopolymers, little inhibitory effect was observed using RT-qPCR analysis.

The diblock copolymers PAMPS₇₅-b-PAaUₙ (with the exception of PAMPSzs-b-PAaUs) and the statistical copolymer P(AMPS₅₀/AaU₅₀) show a strong dose-dependent inhibitory effect against ZIKV. This effect is dependent on the structure of the copolymers and increases with increasing PAaU hydrophobic block length - the highest antiviral efficacy is shown by the PAMPS₇₅-b-PAaU₃₉ block copolymer.

The PAMPS₇₅-b-PAaU₃ block copolymer, characterised by short PAaU hydrophobic blocks and a loose macromolecular conformation, does not show significant antiviral activity under experimental conditions.

The observation, indicating insignificant anti-ZIKV activity of homopolymers and the copolymer with a low content of hydrophobic PAaU units, confirms the role of the macromolecular self-organisation process of PAMPS-PAaU copolymers with the formation of compact core-shell micelles in ZIKV inhibition.

The results indicate that PAMPS-PAaU copolymers (with the exception of PAMPS₇₅-b-PAaUs) are safe and extremely effective ZIKV inhibitors - they have very high selectivity index (SI) values. The results are shown in Table 3.

**Table 3. CC₅₀, IC₅₀ and selectivity index (SI) values of the block copolymers and the PAMPS-PAaU statistical copolymer.**

| **Polymer** | **CC₅₀ [µg/mL]** | **IC₅₀ [µg/mL]** | **SI** |
|---|---|---|---|
| PAMPS₇₅-b-PAaU₃ | 4106 | - | - |
| PAMPS₇₅-b-PAaU₁₂ | 5370 | 2.6 | 2065 |
| PAMPS₇₅-b-PAaU₂₈ | 3580 | 3.2 | 1118 |
| PAMPS₇₅-b-PAaU₃₉ | 3338 | 3.1 | 1107 |
| P(AMPS₅₀/AaU₅₀) | 5461 | 2.6 | 2100 |

### Example 5 - Determination of the mechanism of antiviral action of PAMPS-PAaU copolymers

In order to determine the mechanism of action of the PAMPS-PAaU copolymers and to identify the stage of the replication cycle at which ZIKV infection is inhibited, functional assays were performed as described below at a polymer concentration of 25 µg/mL. The following *in vitro* cell assays were performed:

### 1. Assay of virion inactivation by the studied copolymers.

The aim of this assay was to determine the possibility of virus inactivation by direct interaction of the virus with the studied polymers. A concentrated suspension of ZIKV (TCID₅₀=2 600 000/mL) was incubated for 1 h at room temperature in the presence of copolymers at a concentration of 25 µg/mL. After incubation, samples were diluted 260× with culture medium to achieve a final TCID₅₀ virus concentration of 10 000/mL and copolymer concentration below the active concentration (eliminating the effect of copolymers on the cells themselves). The preparations were analysed using titration according to the method of Reed and Muench¹⁷ by preparing 10 serial five-fold dilutions of the virus and infecting a monolayer of Vero cells. Cells were infected by adding 100 µL of the resulting preparations into the samples and incubated for 2 h at 37°C. The cells were then washed three times with PBS and fresh medium was added. Cells were incubated for 12 h at 37°C. Then, the supernatant was collected and RT-qPCR analysis was performed.

### 2. Assay of cell protection by PAMPS-PAaU

The aim of this assay was to study whether the copolymers interact with host cells and protect them from infection. The effect of copolymers on cells was assessed by incubating Vero cells in medium for 1 h at 37°C in the presence of copolymers (100 µL of copolymer solutions in PBS at 25 µg/mL were added). After this time, the polymer was washed away three times with PBS and the cells were infected with ZIKV (TCID₅₀=10 000/mL, 100 µL) and left for 2 h. The cells were then washed three times with PBS and fresh medium was added. After 12-hour incubation (37°C; 5% CO₂), the supernatant was collected and ZIKV replication analysis was performed by determining the copy number of ZIKV virus RNA using RT-qPCR. Based on the changes in viral replication, the effect of the copolymers on the cells was evaluated.

### 3. PAMPS-PAaU receptor interaction assay

The aim of this assay was to determine the ability of the studied copolymers to block the early stages of viral infection. The effect of the copolymers as inhibitors of the process of virus binding to a receptor on the cell surface was assessed by adsorbing the virus onto Vero cells in the presence of the inhibitor and then removing it from the medium. A 100 µL mixture of the virus with copolymer (TCID₅₀=10 000/mL, 25 µg/mL) was introduced onto cells cooled to 4°C. The control sample contained no copolymer. Samples were incubated for 2 h at 4°C to allow the virus to bind to the receptor while blocking the internalisation (cellular transport was blocked by low temperature). Samples were washed three times with PBS, then supplemented with 100 µL of fresh culture medium and incubated for 12 h (37°C; 5% CO₂). After this time, the supernatant was collected and ZIKV replication analysis was performed using RT-qPCR. Based on the changes in viral replication, the effect of copolymers on virus binding to the cell receptor was evaluated.

### 4. Assay of the effect of PAMPS-PAaU on late stages of infection

The aim of this assay was to assess whether the studied copolymers block the late stages of ZIKV replication. The effect of the copolymers as inhibitors of late stages of infection was assessed by infecting Vero cells without the copolymers, followed by incubation in the presence of copolymers after washing the virus away. Thus, at the very time of virus adsorption onto the cells and its internalisation, the copolymer was not present. In order to allow the virus to enter the cells, they were incubated in medium containing ZIKV (TCID₅₀=10 000/mL, 100 µL) for 2 h at 37°C. After this time, the cells were washed three times with PBS and supplemented with copolymer-supplemented culture medium (100 µL, 25 µg/mL) and incubated for 12 h, which is the duration of a full ZIKV replication cycle (37°C; 5% CO₂). After this time, the supernatant was collected and ZIKV replication analysis was performed using RT-qPCR. Based on the changes in viral replication, the effect of PAMPS-PAaU on the late stages of infection was evaluated.

### Results:

The results showed that the inhibitory effect of the copolymers is related to the fact that the copolymers interfere with the adhesion/binding process of the virus to the cell by effectively competing with the virus for access to the binding site or by changing the charge of the binding site.

### Example 6 - Imaging with a fluorescence confocal microscope

### Confocal microscopy

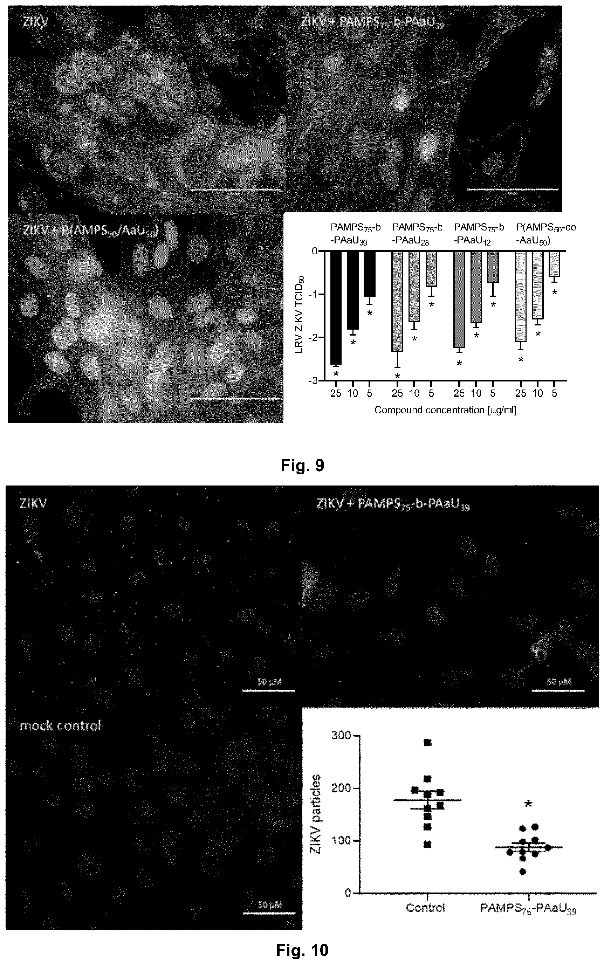

To confirm the above observations, Vero cells were incubated in the absence and presence of the block copolymer PAMPS₇₅-b-PAaU₃₉ or the statistical copolymer P(AMPS₅₀/AaU₅₀) at a concentration of 25 µg/mL at 37°C for 48 h (Fig. 9, usual infection conditions) or at 4°C for 2 h (Fig. 10), allowing adsorption/binding of the virus on the surface, but not allowing it to enter the cell. The cells were then incubated, fixed with 4% formaldehyde, permeabilised with 0.5% Triton X-100 and blocked with a solution of 5% bovine serum albumin. Incubation was carried out for 2 hours with 1 µg/mL rabbit anti-ZIKV Envelope antibody (GeneTex, Irvine, CA, USA). After washing three times with PBS, cells were incubated for 1 hour with 2.5 µg/mL of goat anti-rabbit secondary antibody conjugated with Atto 488 fluorophore and/or with phalloidin conjugated with Alexa Fluor 647 (0.2 U/mL, Invitrogen, Warsaw, Poland) staining cellular actin. Slides were then washed three times with PBS and cell nuclei (DNA) were stained with DAPI (0.1 µg/mL, Sigma-Aldrich, Poznan, Poland) for 20 minutes at room temperature. Cells on the slides were washed several times with PBS and mounted on basal slides using ProLong Diamond Antifade Mountant medium (Life Technologies, Eugene, OR, USA). Fluorescence images were obtained using an EVOS imaging system confocal microscope (Life Technologies, Carlsbad, CA, USA) and processed using Imaged (ver. 1.52b) Fiji software (Madison, WI, USA)¹⁸.

### Results:

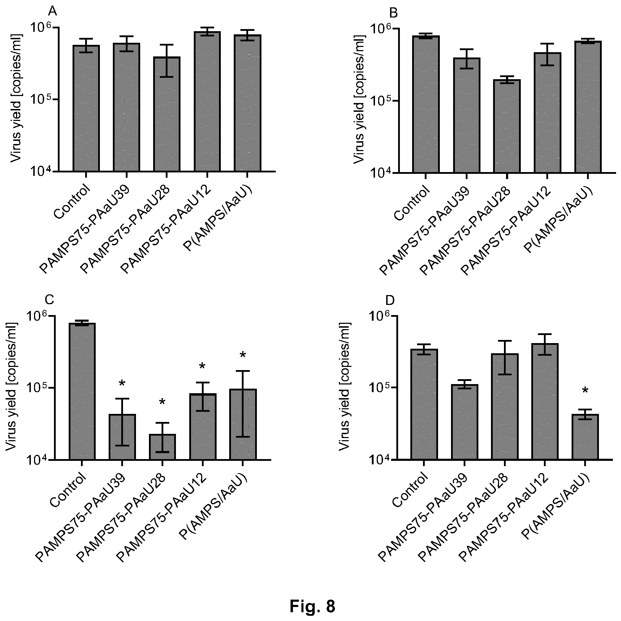

The imaging results confirm strong inhibitory effect of the PAMPS₇₅-b-PAaU₃₉ block copolymer against ZIKV. While the majority of cells used in the control experiment (without polymer) were infected (production of ZIKV virus E protein was observed), only single cells became infected in samples treated with PAMPS₇₅-b-PAaU₃₉ copolymer. The imaging results shown in fig.10 confirm that PAMPS₇₅-b-PAaUₙ copolymers interfere with the ZIKV replication cycle by blocking the site of binding the virus to the host cell and correlate with the results shown in fig.8.

The study found that the most effective were diblock copolymers containing PAMPS ionic blocks of 75 mers and PAaU non-ionic blocks of 12 to 39 mers in their structure. The most effective was the PAMPS₇₅-b-PAaU₃₉ diblock copolymer. Mechanistic assays showed that the studied copolymers block the entry of the virus into the cell by blocking its adhesion to the host cell surface.

The *in vitro* studies have shown that polymers containing anionic groups, as well as hydrophobic aromatic or alkyl substituents, in their structure, such as PAMPS-PAaU copolymers, are extremely promising drug candidates against ZIKV. They exhibit extremely strong inhibitory properties against ZIKV replication in the Vero cell line. Unexpectedly, PAMPS homopolymers were found to have only limited antiviral activity. The PAMPS-PAaU copolymers exhibit very low toxicity to Vero cells (no toxicity at concentrations up to 1 mg/mL, concentration toxic to 50% of cells CC₅₀>3 mg/mL), while having strong antiviral properties against ZIKV (a concentration inhibiting 50% of ZIKV replication (IC₅₀) at about 3 µg/mL). The mechanism of action studies have shown that PAMPS-PAaUs prevent the adhesion of viral particles to the cell, consequently preventing cell infection. Antiviral activity was observed only when the copolymers were present in the medium during infection. Self-organisation of macromolecules in aqueous solutions leading to the formation of highly surface-charged nanostructured micellar systems plays an important role in this process. Analysis of images obtained by fluorescence confocal microscopy showed that when the copolymers were present in the medium during infection, a significant reduction in the number of virus particles on the cell surface is evident.

### Bibliography:

1. Fields, B. N., Knipe, D. M. & Howley, P. M. Fields virology. Fields Virol. (Wolters Kluwer/Lippincott Williams & Wilkins Health, 2013). doi:9781451105636
2. Kraemer, M. U. G., Sinka, M. E., Duda, K. A., Mylne, A., Shearer, F. M., Brady, O. J., Messina, J. P., Barker, C. M., Moore, C. G., Carvalho, R. G., Coelho, G. E., Van Bortel, W., Hendrickx, G., Schaffner, F., Wint, G. R. W., Elyazar, I. R. F., Teng, H.-J. & Hay, S. I. The global compendium of Aedes aegypti and Ae. albopictus occurrence. Sci. data 2, 150035 (2015).
3. Musso, D., Roche, C., Robin, E., Nhan, T., Teissier, A. & Cao-Lormeau, V. M. Potential sexual transmission of zika virus. Emerg. Infect. Dis. 21, 359-361 (2015).
4. Dick, G. W. A. Zika Virus (I). Isolations and serological specificity. 46, 509-520 (1952).
5. MacNamara, F. N. Zika virus: A report on three cases of human infection during an epidemic of jaundice in Nigeria. 48, 139-145 (1954).
6. Duffy, M. R., Chen, T. H., Hancock, W. T., Powers, A. M., Kool, J. L., Lanciotti, R. S., Pretrick, M., Marfel, M., Holzbauer, S., Dubray, C., Guillaumot, L., Griggs, A., Bel, M., Lambert, A. J., Laven, J., Kosoy, O., Panella, A., Biggerstaff, B. J., Fischer, M. & Hayes, E. B. Zika virus outbreak on Yap Island, Federated States of Micronesia. 360, 2536-2543 (2009).
7. Musso, D., Bossin, H., Mallet, H. P., Besnard, M., Broult, J., Baudouin, L., Levi, J. E., Sabino, E. C., Ghawche, F., Lanteri, M. C. & Baud, D. Zika virus in French Polynesia 2013-14: anatomy of a completed outbreak. Lancet Infect. Dis. (2018). doi:10.1016/S1473-3099(17)30446-2
8. Besnard, M., Lastere, S., Teissier, A., Cao-Lormeau, V. M. & Musso, D. Evidence of perinatal transmission of zika virus, French Polynesia, December 2013 and February 2014. Eurosurveillance (2014). doi:10.2807/1560-7917.ES2014.19.13.20751
9. Brasil, P., Pereira, J. P., Moreira, M. E., Nogueira, R. M. R., Damasceno, L., Wakimoto, M., Rabello, R. S., Valderramos, S. G., Halai, U. A., Salles, T. S., Zin, A. A., Horovitz, D., Daltro, P., Boechat, M., Gabaglia, C. R., De Sequeira, P. C., Pilotto, J. H., Medialdea-Carrera, R., Da Cunha, D. C., De Carvalho, L. M. A., Pone, M., Siqueira, A. M., Calvet, G. A., Baiao, A. E. R., Neves, E. S., De Carvalho, P. R. N., Hasue, R. H., Marschik, P. B., Einspieler, C., Janzen, C., Cherry, J. D., De Filippis, A. M. B. & Nielsen-Saines, K. Zika virus infection in pregnant women in Rio de Janeiro. N. Engl. J. Med. (2016). doi:10.1056/NEJMoa1602412
10. Osorio-De-Castro, C. G. S., Miranda, E. S., De Freitas, C. M., De Camargo, K. R. & Cranmer, H. H. The Zika Virus outbreak in Brazil: Knowledge gaps and challenges for risk reduction. Am. J. Public Health (2017). doi:10.2105/AJPH.2017.303705
11. Petersen, E., Wilson, M. E., Touch, S., McCloskey, B., Mwaba, P., Bates, M., Dar, O., Mattes, F., Kidd, M., Ippolito, G., Azhar, E. I., Zumla, A., E., P., M.E., W., S., T., B., M., P., M., M., B., O., D., F., M., M., K., G., I. & E.I., A. Rapid Spread of Zika Virus in The Americas - Implications for Public Health Preparedness for Mass Gatherings at the 2016 Brazil Olympic Games. Int. J. Infect. Dis. 44, 11-15 (2016).
12. Wheeler, A. C. Development of infants with congenital zika syndrome: What do we know and what can we expect? Pediatrics (2018). doi:10.1542/peds.2017-2038D
13. Honein, M. A., Dawson, A. L., Petersen, E. E., Jones, A. M., Lee, E. H., Yazdy, M. M., Ahmad, N., Macdonald, J., Evert, N., Bingham, A., Ellington, S. R., Shapiro-Mendoza, C. K., Oduyebo, T., Fine, A. D., Brown, C. M., Sommer, J. N., Gupta, J., Cavicchia, P., Slavinski, S., White, J. L., Owen, S. M., Petersen, L. R., Boyle, C., Meaney-Delman, D. & Jamieson, D. J. Birth defects among fetuses and infants of US women with evidence of possible zika virus infection during pregnancy. JAMA - J. Am. Med. Assoc. (2017). doi:10.1001/jama.2016.19006
14. Adachi, K. & Nielsen-Saines, K. Zika clinical updates: Implications for pediatrics. Curr. Opin. Pediatr. (2018). doi:10.1097/MOP.0000000000000582
15. Parra, B., Lizarazo, J., Jimenez-Arango, J. A., Zea-Vera, A. F., Gonzalez-Manrique, G., Vargas, J., Angarita, J. A., Zuñiga, G., Lopez-Gonzalez, R., Beltran, C. L., Rizcala, K. H., Morales, M. T., Pacheco, O., Ospina, M. L., Kumar, A., Cornblath, D. R., Muñoz, L. S., Osorio, L., Barreras, P. & Pardo, C. A. Guillain-Barre syndrome associated with Zika virus infection in Colombia. N. Engl. J. Med. (2016). doi:10.1056/NEJMoa1605564
16. Mizusaki, M., Shimada, Y., Morishima, Y. & Yusa, S. I. S.-I. PH-responsive intra- and inter-molecularly micelle formation of anionic diblock copolymer in water. Polymers (Basel). 8, 1-11 (2016).
17. Reed, L. J. & Muench, H. A simple method of estimating fifty per cent endpoints. Am. J. Epidemiol. 27, 493-497 (1938).
18. Schindelin, J., Arganda-Carreras, I., Frise, E., Kaynig, V., Longair, M., Pietzsch, T., Preibisch, S., Rueden, C., Saalfeld, S., Schmid, B., Tinevez, J. Y., White, D. J., Hartenstein, V., Eliceiri, K., Tomancak, P. & Cardona, A. Fiji: An open-source platform for biological-image analysis. Nat. Methods (2012). doi:10.1038/nmeth.2019

## Claims

1. A PAMPS/PAaU copolymer having the formula: 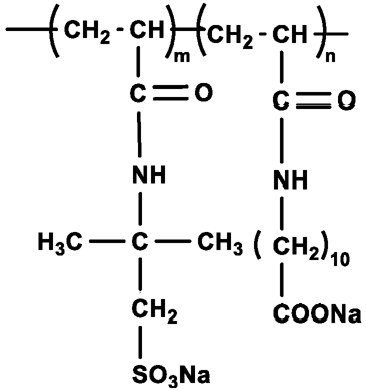 where:
m denotes the length of the PAMPS block, namely the number of AMPS mers, and it is an integer from 40 to 170,
n denotes the length of the PAaU block, namely the number of AaU mers, and it is an integer from 3 to 50,
for use in the treatment and/or prophylaxis of Zika virus infection,
wherein
PAMPS means polymer of sodium salt of 2-acrylamido-2-methyl-1-propanesulfonic acid,
AMPS means a single mer of sodium salt of 2-acrylamido-2-methyl-1-propanesulfonic acid,
PAaU means polymer of sodium 11-(acrylamido)undecanoate,
and AaU means a single mer of sodium 11-(acrylamido)undecanoate.

2. The copolymer for use according to claim 1, **characterised in that** m denotes an integer from 50 to 75, n denotes the length of the PAaU block, namely the number of AaU mers, and it is an integer from 12 to 40.

3. The copolymer for use according to claim 1 and/or claim 2, **characterised in that** it is selected from the group comprising of: the block copolymers PAMPS₇₅-b-PAaU₁₂, PAMPS₇₅-b-PAaU₂₈, PAMPS₇₅-b-PAaU₃₉ and the statistical copolymer P(AMPS₅₀/AaU₅₀),
wherein by block copolymer is meant a copolymer containing two blocks of mers, one block being PAMPS and the other block being PAaU (PAMPSₘ-b-PAaUₙ),
and wherein a statistical copolymer is to be understood as a copolymer in which the mers of AMPS and AaU (PAMPS/PAaU copolymer) are randomly distributed along the chain according to known statistical laws.

4. The copolymer for use according to any of claims 1-3, **characterised in that** it is used at a concentration effective for inhibiting Zika virus replication, preferably between 5 and 25 µg/mL.

5. The copolymer for use according to any of claims 1-4, **characterised in that** the PAMPS₇₅-b-PAaU₃₉ block copolymer is used at a concentration effective for inhibiting Zika virus replication, preferably not less than 5 µg/mL.

## Patentansprüche

1. PAMPS/PAaU-Copolymer mit der Formel: 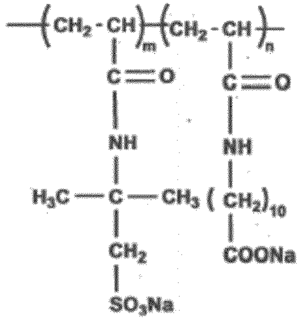 wobei:
m die Länge des PAMPS-Blocks, d.h. die Anzahl der AMPS-mere, bezeichnet und eine ganze Zahl von 40 bis 170 ist,
n die Länge des PAaU-Blocks, d.h. die Anzahl der AaU-mere, bezeichnet und eine ganze Zahl von 3 bis 50 ist,
zur Verwendung bei der Behandlung und/oder Prophylaxe einer Zikavirus-Infektion,
wobei PAMPS ein Polymer des Natriumsalzes von 2-Acrylamido-2-methyl-1-propansulfonsäure bedeutet,
AMPS eine Repetiereinheit des Natriumsalzes von 2-Acrylamido-2-methyl-1-propansulfonsäure bedeutet,
PAaU ein Polymer von Natrium-11-(acrylamido)undecanoat bedeutet und AaU eine Repetiereinheit von Natrium-11-(acrylamido)undecanoat bedeutet.

2. Copolymer zur Verwendung gemäß Anspruch 1, **dadurch gekennzeichnet, dass** m eine ganze Zahl von 50 bis 75 bezeichnet, n die Länge des PAaU-Blocks, d.h. die Anzahl der AaU-mere, bezeichnet und eine ganze Zahl von 12 bis 40 ist.

3. Copolymer zur Verwendung gemäß Anspruch 1 und/oder 2, **dadurch gekennzeichnet, dass** es aus der Gruppe ausgewählt ist, die aus den Blockcopolymeren PAMPS₇₅-b-PAaU₁₂, PAMPS₇₅-b-PAaU₂₈, PAMPS₇₅-b-PAaU₃₉ und dem statistischen Copolymer P(AMPS₅₀/AaU₅₀) besteht,
wobei unter Blockcopolymer ein Copolymer verstanden wird, das zwei Blöcke von Repetiereinheiten enthält, wobei es sich bei einem Block um PAMPS und bei dem anderen Block um PAaU handelt (PAMPSₘ-b-PAaUₙ),
und wobei ein statistisches Copolymer zu verstehen ist als ein Copolymer, bei dem die Repetiereinheiten von AMPS und AaU (PAMPS/PAaU-Copolymer) gemäß bekannten statistischen Gesetzen statistisch entlang der Kette verteilt sind.

4. Copolymer zur Verwendung gemäß einem der Ansprüche 1 bis 3, **dadurch gekennzeichnet, dass** es in einer Konzentration verwendet wird, die effektiv bezüglich der Hemmung der Replikation des Zikavirus ist, vorzugsweise von 5 bis 25 µg/ml.

5. Copolymer zur Verwendung gemäß einem der Ansprüche 1 bis 4, **dadurch gekennzeichnet, dass** das PAMPS₇₅-b-PAaU₃₉-Blockcopolymer in einer Konzentration verwendet wird, die effektiv bezüglich der Hemmung der Replikation des Zikavirus ist, vorzugsweise nicht weniger als 5 µg/ml.

## Revendications

1. Copolymère PAMPS/PAaU répondant à la formule suivante : 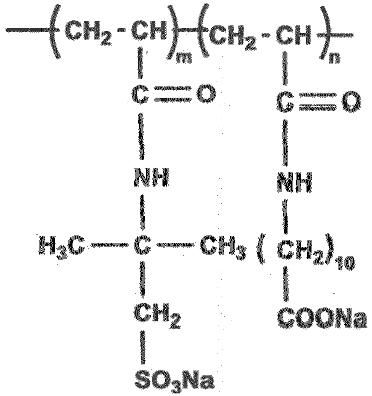 où :
m désigne la longueur du bloc PAMPS, à savoir le nombre de mères AMPS, et c'est un nombre entier de 40 à 170,
n désigne la longueur du bloc PAaU, à savoir le nombre de mères AaU, et c'est un nombre entier de 3 à 50,
pour utilisation dans le traitement et/ou la prophylaxie de l'infection par le virus Zika,
dans lequel
PAMPS signifie polymère de sel de sodium d'acide 2-acrylamido-2-méthyl-1-propanesulfonique,
AMPS signifie un mère simple de sel de sodium d'acide 2-acrylamido-2-méthyl-1-propanesulfonique,
PAaU signifie polymère de 11-(acrylamido)undécanoate de sodium,
et AaU signifie un mère simple de 11-(acrylamido)undécanoate de sodium.

2. Copolymère pour utilisation selon la revendication 1, **caractérisé en ce que** m désigne un nombre entier de 50 à 75, n désigne la longueur du bloc PAaU, à savoir le nombre de mères AaU, et c'est un nombre entier de 12 à 40.

3. Copolymère pour utilisation selon la revendication 1 et/ou la revendication 2, **caractérisé en ce qu'**il est choisi dans le groupe comprenant : les copolymères à blocs PAMPS₇₅-b-PAaU₁₂, PAMPS₇₅-b-PAaU₂₈, PAMPS₇₅-b-PAaU₃₉ et le copolymère statistique P(AMPS₅₀/AaU₅₀),
dans lequel, par copolymère à blocs, on entend un copolymère contenant deux blocs de mères, un bloc étant du PAMPS et l'autre bloc étant du PAaU (PAMPSₘ-b-PAaUₙ),
et dans lequel, un copolymère statistique doit être compris comme un copolymère dans lequel les mères d'AMPS et d'AaU (copolymère PAMPS/PAaU) sont répartis aléatoirement le long de la chaîne selon des lois statistiques connues.

4. Copolymère pour utilisation selon l'une quelconque des revendications 1 à 3, **caractérisé en ce qu'**il est utilisé à une concentration efficace pour inhiber la réplication du virus Zika, de préférence entre 5 et 25 µg/mL.

5. Copolymère pour utilisation selon l'une quelconque des revendications 1 à 4, **caractérisé en ce que** le copolymère à blocs PAMPS₇₅-b-PAaU₃₉ est utilisé à une concentration efficace pour inhiber la réplication du virus Zika, de préférence de pas moins de 5 µg/mL.
